# EUROPEAN PATENT APPLICATION

(11) **EP 1 992 368 A2**
(43) Date of publication of application: **19.11.2008**
(21) Application number: 08007478.4
(22) Date of filing: 17.04.2008
(51) Int. Cl.: A61L 27/00

(54) **Implant material**

(30) Priority: 17.04.2007 JP 2007108304; 15.04.2008 JP 2008105701
(71) Applicant: Kikusui Chemical Industries Co., Ltd., Nagoya-shi Aichi 460-0002 (JP)
(72) Inventor: Kashiwabara, Tateki, Gifu, 509-0103 (JP); Yoshioka, Shigeru, Kanagawa, 236-0031 (JP); Ito, Michio, Nagano, 399-0742 (JP)
(74) Representative: Roth, Klaus

(57) **Abstract**

An implant material includes a base material that includes, as a main component, a partially stabilized zirconia having an average crystal particle diameter of 0.3 µm or less and a porous covering layer that includes ceramic as a main component and has a center pore diameter within a range of 10-100 µm. The covering layer 5 includes 5-10 weight % of calcium.

## Description

### FIELD OF THE INVENTION

The present invention relates to an implant material used for artificial teeth and artificial bones in the fields of dentistry, orthopedic surgery, plastic surgery, oral surgery, etc.

### BACKGROUND OF THE INVENTION

In recent years, a so-called implant technology to restore a lost function by inserting an implant material, such as an artificial tooth root, an artificial bone, etc., into a living body has been drawing attention.

Metal materials, such as titan, have been used as major dental implant materials. However, it is reported that galvanic reaction occurring between an implant material made of titan and an upper structure made of gold alloy causes titan to be eluted, and the eluted titan is distributed in tissues surrounding the implant material. This phenomenon may be considered a trigger of allergy caused by metal ions accumulated in a body.

Accordingly, an implant material made of ceramic which is non-conducting and does not cause galvanic reaction as mentioned above has been proposed (see Japanese Unexamined Patent Application Publication No. 10-85240).

### SUMMARY OF THE INVENTION

An implant material made of ceramic involves a problem that when the implant material is implanted in a living body, it takes a long time to make a bond with tissues of the living body (for example, bone tissues). This is because the pH around the implant material becomes 3-4.5 when the implant material is implanted in the living body, and thus bone growth capacity is reduced.

The present invention, which has been made in view of the above, has an object to provide an implant material capable of making a bond with living tissues in a short time period even in the case of including ceramic as a main component.

(1) In a first aspect of the present invention, an implant material includes: a base material that includes, as a main component, a partially stabilized zirconia having an average crystal particle diameter of 0.3 µm or less; and a porous covering layer that includes ceramic as a main component and has a center pore diameter within a range of 10-100 µm, wherein the covering layer includes 5-10 weight % of calcium.

The implant material of the present invention may achieve an effect, by having the covering layer including calcium, that the implant material can make a bond with living tissues (for example, bone tissues) when implanted in a living body in a short time period.

It may be assumed that this is because the following phenomenon occurs. Specifically, when an implant material made of ceramic is implanted, the pH around tho implant material usually becomes 3-4.5, and thereby growth of living tissues is hindered. However, when the implant material of the present invention is used, calcium ion eluted from the covering layer makes pH neutral. Since neutral pH is a condition that accelerates the growth of living tissues (for example, bone tissues), use of the implant material of the present invention may result in achievement of a bond between the implant material and the living tissues in a short time period.

Especially, in the present invention, the above described effect may be achieved sufficiently since the weight ratio of calcium in the covering layer is 5 weight % or more. Also, weakening of the covering layer may be avoided since the weight ratio is 10 % or less.

Furthermore, the implant material of the present invention may be hardly reduced in toughness (becoming brittle) even when exposed to lactic acid since the base material is constituted by the partially stabilized zirconia having an average crystal particle diameter of 0.3 µm or less.

The average crystal particle diameter of the partially stabilized zirconia is preferably 0.01 µm or more. In the case of 0.01 µm or more, a surface activity will not be excessively high, and thus recrystallization at the time of sintering may be avoided. As a result, the average crystal particle diameter of the partially stabilized zirconia will not become large (for example, 0.3 µm or more). Also, in the case of 0.01 µm or more, a surface area of the partially stabilized zirconia will not be excessively large, and thus the density of the base material may be high at the time of formation and the base material may be densified at the time of sintering.

Since the implant material of the present invention is provided with the porous covering layer, the bond between the implant material and living tissues (for example, bone tissues) may be further stronger when the implant material is implanted in a living body. Specifically, since the living tissues enter into the pores in the layer including calcium, the bond between the implant material and the living tissues may be strengthened.

Especially, in the present invention, since the center pore diameter in the porous material is 10 µm or more, a remarkable effect may be achieved that the bone grows into the pores and thus the implant material is stably-fixed when the implant material is implanted in the living body. Also, since the center pore diameter in the porous material is 100 µm or less, reduction of the surface strength of the implant material may be avoided.

Here, the average crystal particle diameter and the center pore diameter are values measured by a geometric measurement method using an electron microscope (SEM). An example of the partially stabilized zirconia is zirconia partially stabilized by adding yttria. The additive amount of yttria is preferably 2-4 mols, and most preferably 3 mols. An example of the ceramic for forming the covering layer is zirconia.

The implant material of the present invention may be used for artificial teeth and artificial bones in the fields of dentistry, orthopedic surgery, plastic surgery, oral surgery, etc.

The implant material may be provided with a hydroxyapatite layer including hydroxyapatite formed as an upper layer over the covering layer. The implant material may achieve an effect, due to the hydroxyapatite layer, that the implant material is capable of making a bond with living tissues (for example, bone tissues) in a shorter time period when implanted in a living body.

It may be assumed that this is because, when the implant material is implanted in a living body, calcium ion is eluted early from the hydroxyapatite layer and the calcium ion makes pH around the implant material neutral, and thereby accelerates the bond between the implant material and the living tissues (for example, bone tissues).

The hydroxyapatite layer may be a layer including substantially only hydroxyapatite, and also may be a layer including other components in addition to hydroxyapatite to an extent that does not adversely affect the performance of hydroxyapatite.

(2) In a second aspect of the present invention, an implant material includes: a base material that includes, as a main component, a partially stabilized zirconia having an average crystal particle diameter of 0.3 µm or less; a porous covering layer that includes ceramic as a main component and has a center pore diameter within a range of 10-100 µm; and a hydroxyapatite layer including hydroxyapatite formed as an upper layer over the covering layer.

Having the hydroxyapatite layer, the implant material of the present invention may provide an effect that the implant material, when implanted in a living body, can make a bond with living tissues (for example, bone tissues) in a short time period.

It may be assumed that this is because, when the implant material is implanted in a living body, calcium ion is eluted early from the hydroxyapatite layer, and the calcium ion makes pH around the implant material neutral, and thereby accelerates the bond between the implant material and the living tissues (for example, bone tissues).

Since the implant material of the present invention is provided with the porous covering layer, the bond between the implant material and living tissues (for example, bone tissues) may be further stronger when the implant material is implanted in a living body. Specifically, since the living tissues enter into the pores in the layer including calcium, the bond between the implant material and the living tissues may be strengthened.

Especially in the present invention, the fact that the center pore diameter in the porous material is 10 µm or more leads to a remarkable effect that a bone grows into the pores and thus the implant material is stably-fixed when the implant material is implanted in a living body. Also, due to the fact that the center pore diameter in the porous material is 100 µm or less, reduction of the surface strength of the implant material may be avoided.

Furthermore, the implant material of the present invention may be hardly reduced in toughness (becoming brittle) even when exposed to lactic acid since the base material is constituted by the partially stabilized zirconia having an average crystal particle diameter of 0.3 µm or less.

The average crystal particle diameter of the partially stabilized zirconia is preferably 0.01 µm or more. In the case of 0.01 µm or more, a surface activity will not be excessively high, and thus recrystallization at the time of sintering may be avoided.. As a result, the average crystal particle diameter of the partially stabilized zirconia will not become large (for example, 0.3 µm or more). Also, in the case of 0.01 µm or more, a surface area of the partially stabilized zirconia will not be excessively large, and thus the density of the base material may be high at the time of formation and the base material may be densified at the time of sintering.

The hydroxyapatite layer may be a layer including substantially only hydroxyapatite, and also may be a layer including other components in addition to hydroxyapatite to an extent that does not adversely affect the performance of hydroxyapatite.

The partially stabilized zirconia may be obtained by partially stabilizing zirconia by adding, for example, yttria. The additive amount of yttria is preferably 2-4 mols, and most preferably 3 mols.

The ceramic for forming the covering layer may be, for example, zirconia or the like. The ceramic constituting the covering layer is preferably zirconia. The covering layer in the implant material of the present invention includes zirconia as the main component, and thereby achieves a high strength. The zirconia is preferably a zirconia partially stabilized with 3 mols of yttria and having an average crystal particle diameter of 0.3 µm or less. Even when exposed to lactic acid, such a zirconia is hardly reduced in toughness.

The average crystal particle diameter of the zirconia partially stabilized with 3 mols of yttria is preferably 0.01 µm or more. In the case of 0.01 µm or more, a surface activity will not be excessively high, and thus recrystallization at the time of sintering may be avoided. As a result, the crystal particle diameter of the partially stabilized zirconia will not become large (for example, 0.3 µm or more). Also, in the case of 0.01 µm or more, a surface area of the zirconia partially stabilized with 3 mols of yttria will not be excessivoly large, and thus the density of the covering layer may be high at the time of formation and the covering layer may be densified at the time of sintering.

### BRIEF DESCRIPTION OF THE DRAWINGS

One embodiment as the best mode for carrying out the invention will be described below in detail with reference to the accompanying drawings, in which:

FIG. 1 is a front elevation view showing an appearance of an implant body; and

FIG. 2 is a vertical cross-sectional view, including a partial enlarged view, of the implant body along line II-II of FIG. 1.

### BEST MODE FOR CARRYING OUT THE INVENTION

### Embodiment 1

### a) Constitution of Implant Body

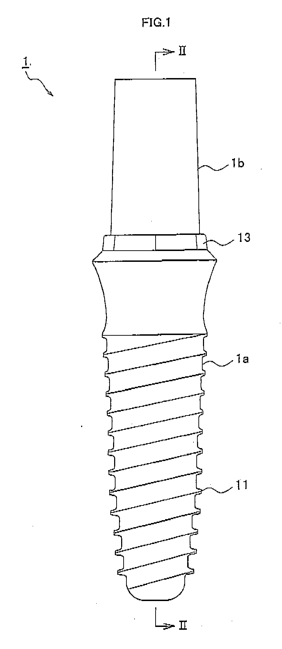

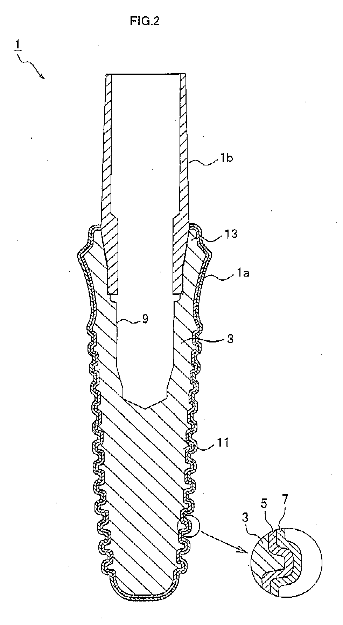

A constitution of a dental implant body 1 will be described based on FIG. 1 and FIG. 2.

The implant body 1 includes an implant portion 1a to be implanted in a living body and an exposed portion 1b which is exposed from the living body and to which an upper structure (not shown) is mounted. The implant portion 1a has a bar-like configuration with a hole 9 extending downward from an upper end of the implant portion 1a. In an area of an outer circumferential surface, including a lower end, of the implant portion 1a, a screw groove 11 to implant the implant portion 1a into the living body is formed. Further, in a vicinity of the upper end, a nut portion 13 having a hexagonal cross-section is formed in the outer circumferential surface of the implant portion 1a. By attaching a spanner wrench to the nut portion 13 and rotating the implant portion 1a, the implant portion 1a may be screwed into the living body.

The implant portion 1a includes a base material 3, a covering layer 5 and a hydroxyapatite layer 7. The base material 3 is made of dense zirconia. The dense zirconia is a partially stabilized zirconia including 3 mols of yttria and having an average crystal particle diameter of 0.3 µm. The covering layer 5 is a layer covering a surface of the base material 3. The covering layer 5 includes, as a main component, the same dense zirconia as in the base material 3 and also includes 5 weight % of calcium. The covering layer 5 is a porous layer having a center pore diameter of 50 µm. The hydroxyapatite layer 7, which is a layer made of hydroxyapatite, further covers the covering layer 5 from outside.

The exposed portion 1b is a tubular member having a penetrating hole extending from an upper end to a lower end thereof. A part of the exposed portion 1b including the lower end thereof is inserted into the hole 9 of the implant portion 1a. The exposed portion 1b is made of a partially stabilized zirconia including 3 mols of yttria and having an average crystal particle diameter of 0.3 µm in a same manner as the base material 3.

### b) Manufacturing Method of Implant Body 1

### (i) Manufacturing Method of Implant Portion 1a

A hollow cylindrical 3 mol yttria-stabilized zirconia ceramic compact was formed by a powder press method. Then, the compact was fired at 1300 °C in a furnace to obtain a sintered ceramic body. The sintered ceramic body was machine processed into a configuration shown in FIG. 1 and FIG. 2 with a machining center having a diamond tool as a grinding jig to obtain the base material 3. An average crystal particle diameter of the zirconia in the base material 3 was 0.3 µm.

Next, a slurry was dip coated on an outer circumferential surface of the base material 3. The slurry includes powder of the same dense zirconia as the dense zirconia constituting the base material 3 and calcium carbonate powder having an average center particle diameter of 2 µm as a foaming agent, at a volume ratio of these powders of 90:10. After the slurry was dip coated, drying for one day was performed and then firing at 1300 °C was performed. As a result, the covering layer 5 of dense zirconia having a center pore diameter of 50 µm and a pore rate of 15 % was formed. The covering layer 5 includes pores formed by carbon dioxide produced by foaming of the foaming agent and 5 parts by weight of calcium against 100 parts by weight of the entire covering layer 5. The thickness of the covering layer 5 was 2·10 µm. The thickness of the covering layer 5 is adjustable by the amount of the slurry to be applied and the concentration of the slurry.

Spherical carbon powder as a burning agent may be used in the slurry in place of calcium carbonate powder, in order to form the covering layer 5. In this case, the covering layer 5 may be formed by impregnating calcium into a porous layer produced after firing.

Subsequently, an operation of applying an alcohol solution including calcium 2-ethylhexanoic acid and tricresyl phosphate to the outer circumferential surface of the base material 3, heating up to 400 °C and firing for 10 minutes was repeated four times to form the hydroxyapatite layer 7. Thus, the implant portion 1a was completed.

### (ii) Manufacturing Method of Exposed Portion 1b

The exposed portion 1b was manufactured in the same manner as the Manufacturing method of the base material 3 in the implant portion 1a. Specifically, a hollow cylindrical 3 mol yttria-stabilized zirconia ceramic compact was formed by a powder press method, the compact was fired at 1300 °C in a furnace to obtain a sintered ceramic body, and the sintered ceramic body was machine processed into a configuration of the exposed portion 1b shown in FIG. 1 and FIG. 2 with a machining center having a diamond tool as a grinding jig.

### (iii) Jointing of Implant Portion 1a and Exposed Portion 1b

The lower end of the exposed portion 1b was inserted into the hole 9 of the implant portion 1a, and thus the implant body 1 was completed.

### Embodiment 2

The implant body 1 was manufactured in basically the same manner as in Embodiment 1. In Embodiment 2, however, a porous layer of dense zirconia not including calcium was formed in place of the covering layer 5. The porous layer was formed by performing the same process for forming the covering layer 5 as in Embodiment 1 up to the step of firing and without performing calcium impregnation.

### Embodiment 3

The implant body 1 was manufactured in basically the same manner as in Embodiment 1. In Embodiment 3, however, the hydroxyapatite layer 7 was not formed.

### Embodiment 4

The implant body 1 was manufactured in basically the same manner as in Embodiment 1. In Embodiment 4, however, the covering layer 5 has a center pore diameter of 10 µm. The center pore diameter can be achieved by setting the average particle diameter of the burning agent included in the slurry to be used for forming the covering layer 5 to 14 µm. The reason that average particle diameter of the burning agent is set to 14 µm larger than the center pore diameter of 10 µm is that contraction occurs during the step of firing.

### Embodiment 5

The implant body 1 was manufactured in basically the same manner as in Embodiment 1. In Embodiment 5, however, a porous layer of dense zirconia not including calcium was formed in place of the covering layer 5. The porous layer was formed by performing the same process for forming the covering layer 5 as in Embodiment 1 up to the step of firing and without performing calcium impregnation. Also, the center pore diameter of in the porous layer was set to 10 µm.

### Embodiment 6

The implant body 1 was manufactured in basically the same manner as in Embodiment 1. In Embodiment 6, however, the average crystal particle diameter of partially stabilized zirconia including 3 mols of yttria and constituting the base material 3 was set to 0.01 µm.

Constitutions of the implant bodies 1 manufactured in Embodiments 1-6 are shown in Table 1.

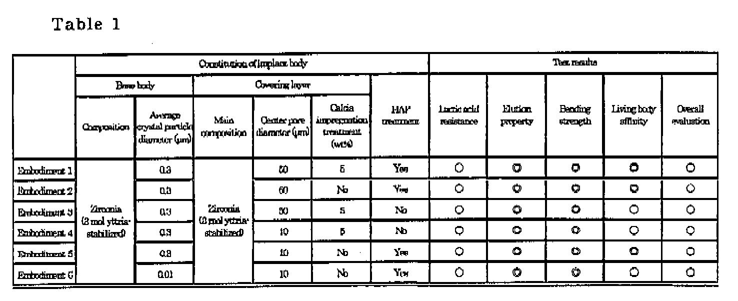

### (Comparison Examples)

Implant bodies 1 in Comparison examples 1-16 were manufactured so as to be basically the same but partially modified as compared with Embodiment 1. The respective constitutions are shown in Table 2.

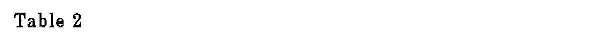

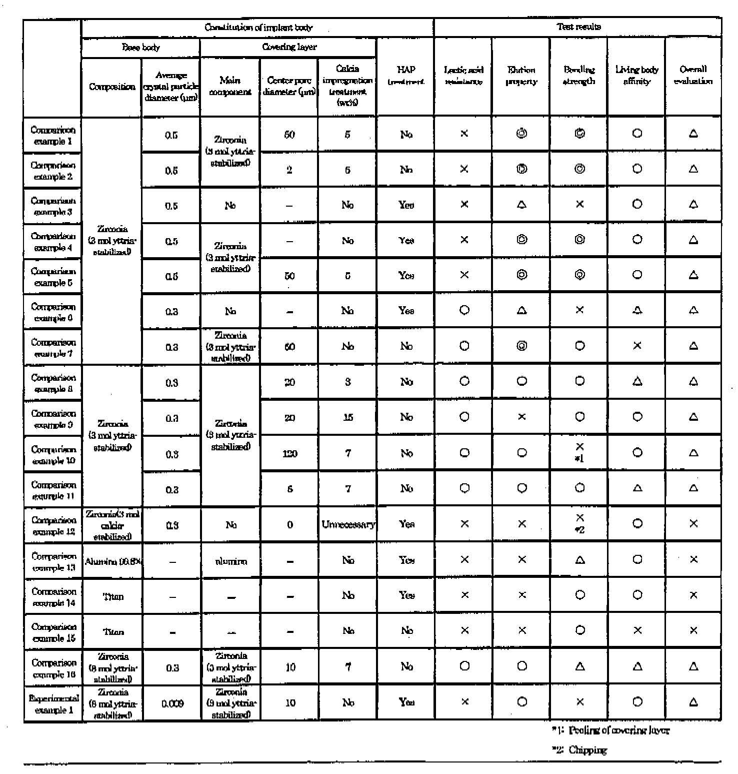

As shown in Table 2, the average crystal particle diameter of the dense zirconia constituting the base material 3 and the exposed portion 1b was set to 0.5 µm in Comparison examples 1-5. A covering layer was not formed in Comparison examples 3, 6, 12, 14 and 15. Accordingly, "the covering layer is porous" or "the covering layer includes 5-10 weight % of calcium" is of course not applicable to these Comparison examples. In Comparison examples 4 and 13, the covering layer was formed to be a uniformly filled layer instead of a porous layer. This may be achieved by not adding any burning agent to the slurry for forming the covering layer. In Comparison examples 2, 8-11 and 16, the center pore diameter of the covering layer was set to a value different from 50 µm. In Comparison examples 4, 7 and 13, the covering layer was not impregnated with calcium. In Comparison examples 8-11 and 16, the amount of calcium included in the covering layer was set to a value different from 5 weight %.

In Comparison examples 1-2, 7, 8-11, 15 and 16, the hydroxyapatite layer 7 was not formed. In Comparison example 13, the base material and the exposed portion were made of alumina. Specifically, the base material and the exposed portion used in Comparison example 13 were obtained by forming a 99.8 % alumina compact by a powder press method, firing the compact at 1600 °C in a furnace to obtain an alumina sintered body, and machine processing the alumina sintered body into the same configurations as those of the base material 3 and the exposed portion 1b in Embodiment 1. In Comparison example 13, the covering layer was also made of alumina. The covering layer was obtained by applying a slurry including alumina powder to a surface of the base material and firing at 1600 °C.

In Comparison examples 14-15, the base material and the exposed portion were made of titan. Specifically, the base material used in Comparison examples 14-15 was obtained by machine processing a titan wire rod into the same configurations as the base material 3 and the exposed portion 1b in Embodiment 1.

In Comparison example 16, the base material was made of completely stabilized zirconia to which 8 mols of yttria was added.

Also, the implant body 1 in Experimental example 1 was manufactured so as to be basically the same but partially modified as compared with Embodiment 5. The constitution is shown in Table 2. In Experimental example 1, the average crystal particle diameter of partially stabilized zirconia including 3 mols of yttria was set to 0.009 µm.

### (Tests to ascertain effects of the invention)

The following tests were performed using, as test subjects, the implant bodies 1 manufactured in the embodiments, the comparison examples and the experimental example, and implant bodies manufactured in the same manner as in the above embodiments and examples but having different configurations.

(i) Bending Strength Test and Lactic Acid Resistance Test

Test subjects, each having a width of 5 mm, a length of 30 mm and a thickness of 1 mm, manufactured in the same manner as the implant portions 1a of the implant bodies 1 in the respective embodiments and comparison examples were prepared. The bending strength of the test subjects was measured using a universal testing machine (INSTORON5882, by Instron) under the conditions of a cross head speed of 0.5 mm/min. and a support span of 20 mm. The bending strength was evaluated according to the following criteria depending on the value of the bending strength.
⊚: more than 800 MPa
○: 600-800 MPa
△: 300-600 MPa
×: less than 300 MPa

(ii) Lactic Acid Resistance Test

After immersing test subjects formed in a same manner as the test subjects whose bending strengths were measured in the above (i), in a lactic acid solution with a concentration of 1 % for five months, bending test was performed in a same manner again. The lactic acid resistance was evaluated according to the following criteria depending on the value of the bending strength after the immersion against 100 for the bending strength before the immersion.
○: the bending strength after the immersion is within a range of 95-105
△: the bending strength after the immersion is within a range of 60-94
×: the bending strength after the immersion is less than 60

(iii) Elution Property Test

The implant portion 1a in each of the implant bodies 1 manufactured in the embodiments and comparison examples was immersed in 70 ml of lactic acid solution with a concentration of 1 % for five months. Then, components (zirconia, alumina and titan) of the base material 3 eluted into the solution were detected using a plasma emission spectrometer (ICPS-7510, by Shimadzu Corporation). Subsequently, the elution property was evaluated according to the following criteria depending on the eluted amount.
⊚: elution concentration is 0.5 ppm or less
○: elution concentration is 0.5 to 1.0 ppm
△: elution concentration is 1.0 to 10 ppm
×: elution concentration is more than 10 ppm

(iv) Living Body Affinity Test

The implant portion 1a was implanted in a rat tibia bone, and a state of contact between the implant portion 1a and the bone was measured after four weeks. Then, the living body affinity was evaluated according to the following criteria.
⊚: 90 to 100 % contact with bone
○: 70 to 90 % contact with bone
△: 50 to 70 % contact with bone
×: less than 50 % contact with bone

The results of the tests are shown in Table 1 and Table 2. The implant bodies in Embodiments 1-6 are superior in any of the tests, as shown in Table 1 and Table 2.

In contrast, the lactic acid resistance is inferior in Comparison examples 1-5 since the average crystal particle diameter of the dense zirconia constituting the base material 3 is as large as 0.5 µm. In Comparison examples 3, 6 and 12, the bending strength is low since a porous covering layer is not provided. In Comparison example 7, the living body affinity is inferior since the covering layer is not impregnated with calcium and a hydroxyapatite layer is not formed. In Comparison example 8, the living body affinity is inferior since the covering layer has a low calcium content and a hydroxyapatite layer is not formed. In Comparison example 9, the elution property is inferior since the covering layer has a too high calcium content. In Comparison example 10, the bending strength is low since the covering layer has a too large center pore diameter. In Comparison example 11, the living body affinity is inferior since the covering layer has a too small center pore diameter. In Comparison example 13-15, the lactic acid resistance and the elution property are inferior since the base material is made of alumina or titan. In Comparison example 15, the living body affinity is inferior since a covering layer including calcium or a hydroxyapatite is not formed. In Comparison example 16, the bending strength and the living body affinity are slightly inferior sinco the base material is made of completely stabilized zirconia. In Experimental example 1, the lactic acid resistance and the bending strength are slightly inferior since the average crystal particle diameter of partially stabilized zirconia including 3 mols of yttria which constitutes the base material 3 is 0.009 µm.

It is not to be mentioned that the present invention should not be limited to the above described embodiments, but may be embodied in various forms without departing from the present invention.

For example, the implant body 1 may be constituted by the implant portion 1a and the exposed portion 1B formed integrally with each other. In this case, the covering layer 5 and the hydroxyapatite layer may be formed all over the outer circumferential surface of the implant body 1 or may be formed only in a region corresponding to the implant portion 1a.

## Claims

1. An implant material, comprising:
a base material that includes, as a main component, a partially stabilized zirconia having an average crystal particle diameter of 0.3 µm or less; and
a porous covering layer that includes ceramic as a main component and has a center pore diameter within a range of 10-100 µm,
wherein the covering layer includes 5-10 weight % of calcium.

2. The implant material according to claim 1, further comprising a hydroxyapatite layer including hydroxyapatite and formed as an upper layer over the covering layer.

3. An implant material, comprising:
a base material that includes, as a main component, a partially stabilized zirconia having an average crystal particle diameter of 0.3 µm or less;
a porous covering layer that includes ceramic as a main component and has a center pore diameter within a range of 10-100 µm; and
a hydroxyapatite layer including hydroxyapatite and formed as an upper layer over the covering layer.

4. The implant material according to claim 1, wherein the ceramic constituting the covering layer is zirconia.

5. The implant material according to claim 3, wherein the ceramic constituting the covering layer is zirconia.
